# EUROPEAN PATENT SPECIFICATION

(11) **EP 1 621 188 B1**
(45) Date of publication and mention of the grant of the patent: **10.07.2013**
(21) Application number: 04731709.4
(22) Date of filing: 07.05.2004
(51) Int. Cl.: A61K 9/70, A61K 47/22, A61K 47/32, A61K 45/00, A61K 31/192, A61P 29/00

(54) **ULTRAVIOLET-SHIELDING TYPE PATCH**
PFLASTER VOM ULTRAVIOLET-SCHUTZTYP
PATCH ANTI-ULTRAVIOLETS

(30) Priority: 07.05.2003 JP 2003128934
(43) Date of publication of application: 01.02.2006
(73) Proprietor: Hisamitsu Pharmaceutical Co., Inc., Tosu-shi Saga 841-0017 (JP)
(72) Inventor: HASHIMOTO, Yoshiaki, HISAMITSU PHARMAC. CO., INC., Tosu-shi, Saga 841-0017 (JP); TAKADA, Yasunori, HISAMITSU PHARMAC. CO., INC., Tosu-shi, Saga 841-0017 (JP); TSURUDA, Kiyomi, HISAMITSU PHARMAC. CO., INC, Tosu-shi, Saga 841-0017 (JP)
(74) Representative: Gassner, Wolfgang
(86) International application number: PCT/JP2004/006024
(87) International publication number: WO 2004/098575

(56) References cited:
- EP-A1- 0 698 393
- EP-A1- 0 782 861
- EP-A1- 1 179 558
- EP-A1- 1 197 209
- EP-A1- 1 238 664
- EP-A1- 1 269 999
- WO-A1-01/05381
- WO-A1-01/43729
- WO-A1-01/68061
- WO-A1-96/11022
- WO-A1-98/24423
- JP-A- 8 053 354
- JP-A- 8 133 964
- JP-A- 2000 281 821
- JP-A- 2001 039 864
- JP-A- 2002 022 956
- DATABASE WPI Thomson Scientific, London, GB; AN 1985-239345 XP002536942, -& JP 60 155111 A 15 August 1985 (1985-08-15)
- RADSCHUWEIT ET AL: "UV-induces formation of hydrogen peroxide based on the photochemistry of ketoprofen", PHOTOCHEMISTRY AND PHOTOBIOLOGY, WILEY-BLACKWELL PUBLISHING, INC, US, vol. 73, no. 2, 1 February 2001 (2001-02-01), pages 119-127, XP002985755, ISSN: 0031-8655, DOI: 10.1562/0031-8655(2001)073&LT,0119:UIFOHP& GT,2.0.CO,2

## Description

### [Detailed Description of the Invention]

### [Technical Field]

The invention relates to a patch which is excellent in ultraviolet-shielding effect.

### [Background Art]

Since a patch is used by sticking it on a skin surface, in outdoors there is a case that it receives irradiation of sunrays depending on its application part. Therefore, in a patch containing a compound which is easily decomposed by ultraviolet from the sun, there are problems in which an original drug effect cannot be exhibited due to decomposition of a drug in base or its photodecomposition products induce a side effect or the like.

Conventionally, as means to avoid such side effects due to ultraviolet, it has generally been tried to add an ultraviolet absorbent in a base. For example, although there was an external preparation to suppress the photodedecomposition of an efficacious ingredient by blending an ultraviolet absorbent in a base (ex. see Patent document 1), there was a problem that the safety was feared because the ultraviolet absorbent itself was contacted and absorbed. In addition, a method to contain an ultraviolet absorbent in a backing of a patch has been proposed (ex. see Patent documents 2 and 3). There is a percutaneous absorption patch having a laminate as a backing, which consists of not less than two layers, and consisting of a resin film in which at least one layer of said laminate contains an ultraviolet absorbent (ex. see Patent document 4). Further, a patch is disclosed in which the photo-stability of a drug is increased by a monolayer backing treated with an organic ultraviolet absorbent and/or an inorganic ultraviolet absorbent (ex. see
Patent document 5).

However, although in these the effect to shield ultraviolet ray irradiated from artificial lights such as a mercury lamp and a room light is recognized, these are insufficient to suppress enough the photodecomposition of a drug occurring in case of being exposed to the direct rays of the sun in a season when an ultraviolet dose is high, and further, there were the demerits that auxiliary devices such as covering a patch by clothing or by a protecting cover was necessary.
Patent document 1: JP, B, 5-8169
Patent document 2: JP, B, 3-76285
Patent document 3: JP, U, 5-30118
Patent document 4: JP, A, 10-265371
Patent document 5: WO 01/68061

### [Disclosure of the Invention]

### [Problems to Be Solved by the Invention]

Consequently, the object of the invention is to provide patches which do not cause photodecomposition of drugs even when exposed to the direct rays of the sun in a season of high ultraviolet dose and which exhibit satisfactory drug effects and are excellentin physical properties and stability of pharmaceutical preparations.

### [Means to Solve the Problems]

During extensive research to solve the above problems, the inventors found that by an ultraviolet shielding treatment of a polyester-stretching backing with a specific hydroxyphenylbenzotriazole derivative, photodecomposition of a drug in a patch can extremely be suppressed and completed the invention.

Namely, the invention relates to a patch comprising a polyester backing and a pressure-sensitive adhesive layer formed on one surface of the backing and containing a nonsteroidal anti-inflammatory drug (NSAID) wherein the backing contains a hydroxyphenylbenzotriazole derivative represented by the general formula (1): 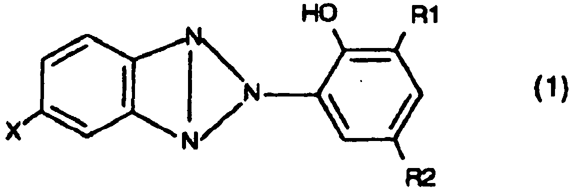
(1) wherein R₁ and R₂ are each independently hydrogen or C₁₋₈ alkyl; and X is halogen atom.

In addition, the invention relates to the patch, wherein the backing contains further titanium oxide.

Further, the invention relates to the patch, wherein the ultraviolet transmittance of the backing is not more than 2%.

In addition, the invention relates to the patch, wherein the weight of the backing is 100g/m² - 130g/m².

Further, the invention relates to the patch, wherein the nonsteroidal anti-inflammatory drug (NSAID) is ketoprofen.

In addition, the invention relates to the patch, wherein the pressure-sensitive adhesive layer consists of a styrene-isoprene-styrene block copolymer and/or polyisobutylene.

Further, the invention relates to the patch, wherein the pressure-sensitive adhesive layer contains no ultraviolet absorbent.

### [Effect of the Invention]

By treatment of a polyester backing with a specific benzotriazole ultraviolet absorbent, a patch of the invention can surprisingly lower the ultraviolet transmittance of the backing, exhibiting satisfactory drug effects even when exposed to the direct rays of the sun in a season of high ultraviolet dose. Therefore, it can favorably be used for a nonsteroidal anti-inflammatory drug (NSAID) which is low in stability toward ultraviolet. In addition, even when a pressure-sensitive adhesive layer contains no ultraviolet absorbent, it has no degradation of the pressure-sensitive adhesive layer and is excellent in stability and also is excellent in safety due to no contact to the skin of an ultraviolet absorbent itself.

In a patch of the invention, the ultraviolet transmittance of a backing is preferably not more than 2% under the condition that the ultraviolet intensity is 3 mW/cm². Further, as to the above ultraviolet dose, change by a season and area is recogni zed, and as one example, a daytime ultraviolet intensity on August 22, 2002 in Tosu-shi, Saga prefecture was measured, showing 2.9mW/cm² and 81936mJ/cm² of the accumulated ultraviolet dose per day, and to the contrary the ultraviolet intensity in the same area on November 5, 2002 was 1.6 mW/cm² and 47340mJ/cm² of the accumulated ultraviolet dose. That is, the patch of the invention can unusually lower the ultraviolet transmittance even under the strong ultraviolet intensity in a season of high ultraviolet dose.

### [Best Embodiment for Carrying out the Invention]

The patch of the invention can be used mainly as a plaster (a tape preparation).

The patch of the invention is made to contain a benzotriazole ultraviolet absorbent which is adsorbed, absorbed or fixed to a polyester backing, or is incorporated in fibers consisting of the backing. The benzotriazole ultraviolet absorbent on the above invention is a benzotriazole derivative represented by the formula (1), wherein R₁ and R₂ represent hydrogen or lower alkyl groups, preferably C₁-C₈ alkyl groups in view of easy treatment of the polyester backing, more preferably methyl or t-butyl.

X represents a halogen atom, preferably fluorine, bromine or chlorine, in particular preferably chlorine in view of easy treatment of the polyester backing.

In addition, R₁ and R₂ may be same or different.

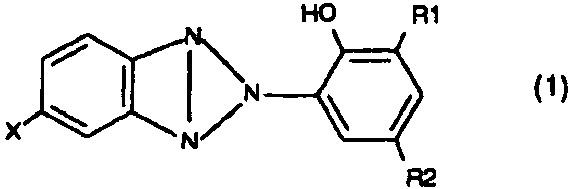

Specifically, the following compounds are illustrated. That is, illustrative are 2-(3-t-butyl-5-methyl-2-hydroxyphenyl)-5-chlorobenzotriazol e, 2-(3,5-di-t-butyl-2-hydroxyphenyl)-5-chlorobenzotriazole, etc.

As for an ultraviolet shielding treatment using these benzotriazole ultraviolet absorbents, a method to adsorb, absorb or fix said absorbents to fibers or cloth is used, which are the material of a backing for the patch, consisting of monolayer. In addition, in a production step of fibers which are the above backing material (polymerization or fiber forming steps), polymer is reformed by addition or incorporation with said absorbent, and then the reformed polymer may be used as a material for the backing by fiber formation.

The content of these benzotriazole ultraviolet absorbents in a backing is preferably 0.01-20% by mass against the total mass of the backing (containing the absorbent), more preferably 0.05-5% by mass, further preferably 1-2% by mass.

Further, in order to obtain an ultraviolet-shielding effect of the backing used in the invention, it may be treated with a metal oxide which is an ultraviolet-shielding agent, and specifically, one or more species selected from titanium oxide, zinc oxide, ferric oxide, talc, kaolin, alumina and calcium carbonate can be blended. In particular, treatment using titanium oxide is preferable.

As an ultraviolet screening treatment using these inorganic shielding agents, a method of fiber formation after reforming by addition or incorporation of the inorganic shielding agents into polymer is generally used in the production step of fibers (polymerization or fiber forming steps).

The content is preferably 0.1-20% by mass against the total mass of the backing, more preferably 0.5-10% by mass, and the shielding effect toward ultraviolet can satisfactorily be exhibited by these blend ratios.

The material of the backing is a polyester cloth, and specifically, illustrative are polyethylene terephthalate, etc. These can be used by treatment into woven fabric, knitted fabric, non-woven fabric, film or the like.

The weight of the backing is preferably 100g/m²-130g/m², more preferably 105g/m²-120g/m² considering the transmittance of ultraviolet and use feeling toward the skin.

The ultraviolet transmittance of the backing used in the invention is preferably not more than 2.0%, more preferably not more than 1.5%, further preferably not more than 1.0% under the condition of 3.0 mW/cm² of ultraviolet intensity. Further, as for measurement of the ultraviolet intensity, an ultraviolet intensity meter (Topcon Co., Ltd., UVR-2) is used, and UD-36 is used for a receiving part, whereby the measuring wave length is 310-400nm. As for the calculation of the ultraviolet transmittance, an ultraviolet dose transmitting through the backing is measured under a circumstance in which a direct sunlight irradiates enough to the backing, and the ultraviolet intensity without the above preparation is made 100, calculating each transmittance.

As for the nonsteroidal anti-inflammatory drugs (NSAID) used in the patch of the invention, ketoprofen, diclofenac, suprofen, piroxicam, indomethacin, flurbiprofen, felbinac, loxoprofen, ibuprofen, ketorolac, naproxen, benoxaprofen, carprofen, fenoprofen, or salts thereof, and one or more of these drugs can be blended. Among these nonsteroidal anti-inflammatory drugs, ketoprofen is most appropriate. The blend ratio of the nonsteroidal anti-inflammatory drug is 0.01-30% by mass based on the total amount of the base containing the drug, preferably 0.1-16% by mass, including a form of a medically acceptable inorganic salt or organic salt, and satisfactory drug effects can be expected by this blend ratio.

As for the base used in the patch of the invention, a rubber base is preferable, and as a rubber base, illustrative are polyisoprene rubber, polyisobutylene rubber, natural rubber, styrene-butadiene-styrene block copolymer, styrene-isoprene-styrene block copolymer, styrene-butadiene block copolymer, styrene-isoprene block copolymer, styrene-isoprene-butadiene block copolymer, styrene-ethylene-propylene-styrene block copolymer and the like as synthetic rubbers or natural rubbers.

Further, since styrene-isoprene-styrene block copolymer is easily degradable against ultraviolet and is poor in photo-stability, the backing used in the invention can be favorably used. In case of using styrene-isoprene-styreneblock copolymer, the average molecular weight is preferably 100,000-300,000, and illustrative are, for example, KRATON D-KX401CS or D-1107CU manufactured by (Shell Chemical Co., Ltd.), SIS-5000 or SIS-5002 (manufactured by JSR Co., Ltd.), Quintac 3530, 3421 or 3570C (Zeon Co., Ltd.) and Solprene 428 (PHILLIPS PETROLEUM Co., Ltd.). As for the base, one or more of these styrene-isoprene-styrene block copolymers can be blended, and the content is preferably 10-50% by mass based on the total amount of the base considering the agglutinative strength and workability, more preferably 13-40% by mass, further preferably 15-30% by mass.

As to the base of the patch of the invention, sticking properties to the skin, a pain at the time of release, a rash on the skin and the like are greatly improved by containing styrene-isoprene-styrene block copolymer with the above average molecular weight at the above blend ratio and more preferably by adjusting further the viscosity and the adhesive strength.

In addition, the base of the patch of the invention may be blended with polyisobutylene, and the content is preferably 1-50% by mass based on the total amount of the base, more preferably 5-40% by mass. Further, two or more species of polyisobutylenes with a different average molecular weight may be used in combination, and,for example, the combination of polyisobutylene of viscosity average molecular weight (Staudinger method) of 5,000-15,000 and polyisobutylene of viscosity average molecular weight of 50,000-200,000 is preferable. Furthermore, blending of these polyisobutylenes at a specific ratio is further preferable.

As polyisobtylene of viscosity average molecular weight of 5,000-15,000 illustrative are Vistanex LM-MS and LM-MH (manufactured by Exxon Chemical Co., Ltd.), Tetrax 4T, 5T and 6T (manufactured by Nihon Sekyu Kagaku Co., Ltd.), Oppanol B12SF and B15SF (manufactured by BASF Japan Co., Ltd.), etc., and one or more of these can be blended in a base of a tape preparation. The content is preferably 1-50% by mass based on the total amount of the base, more preferably 5-30% by mass.

As polyisobtylene of viscosity average molecular weight of 50,000-200,000 illustrative are Vistanex MML-80, MML-100, MML-120 and MML-140 (manufactured by Exxon Chemical Co., Ltd.), Oppanol B-80, B-100, B-120 and B-150 (manufactured by BASF Japan Co., Ltd.), etc., and one or more of these can be blended in a base of a tape preparation. The content is preferably 0.1-40% by mass based on the total amount of the base, more preferably 1-30% by mass, and by these blend ratio and more preferably further by adjustment of the viscosity and the adhesive strength, the adhesive strength of the base, a long time sticking properties for the skin, a pain at the time of release, a rash on the skin and the like can greatly be improved.

Further, when two or more of polyisobutylenes with different viscosity average molecular weight are blended, it is preferable that the total amount of the polyisobutylenes does not exceed 50% by mass based on the total amount of the base.

A preferable adhesive base related to the invention contains a styrene-isoprene-styrene block copolymer, polyisobutylene, a tackifier and a plasticizer, and after the styrene-isoprene-styrene block copolymer, polyisobutylene and the tackifier are mixed at a desirable ratio, this mixture is adjusted to have the above viscosity by the plasticizer to obtain it. The adhesive strength of the patch of the invention can be adjusted mainly by adjusting the composition of the adhesive base.

As tackifiers, preferably those with softening point of 60°C-150°C, for example, rosin esters,hydrogenated rosin esters, maleic acid degenerated rosin esters, polyterpene resins and petroleum resins can be used, and specifically, Ester Gum A, AA-G, H or HP (manufactured by Arakawa Chemical Industris, Ltd.), Hariester L, S or P (manufactured by Harima Chemicals Inc.), Pinecrystal KE-100 or KE-311 (manufactured by Arakawa Kagaku Co., Ltd.), HercolynD (manufactured by Rika Hercules Co. , Ltd.), Foral 85 or 105 (manufactured by Rika Hercules Co., Ltd.), Staybelite Ester 7 or 10 (manufactured by Rika Hercules Co., Ltd.), Pentalyn 4820 or 4740 (manufactured by Rika Hercules Co., Ltd.), ARKON P-85 or P-100 (manufactured by Arakawa Kagaku Co., Ltd.), Escorez 5300 (manufactured by Exxon Chemical Co., Ltd.), Clearon K, M or P (Yasuhara Chemical Co., Ltd.) and the like are illustrated, and one or more of these can be blended in the adhesive base. The content of a tackifier is preferably 5-50% by mass based on the total amount of the base, more preferably 7-45% by mass, further preferably 10-40% by mass, wherein the viscosity and adhesive strength are adjusted in the above range. By this blend ratio are greatly improved the adhesive strength of the obtained base, sticking properties to the skin, a pain at the time of release, a rash on the skin and the like.

As plasticizers, preferably those with solution viscosity of 10-100 centistokes (40°C), for example, almond oil, olive oil, camellia oil, persic oil, peanut oil, olefin acid and liquid paraffin are illustrated, and one or more of these can be blended in the adhesive base. The blend ratio of a plasticizer is preferably 10-70% by mass based on the total amount of the base, more preferably 15-60% by mass, further preferably 20-55% by mass, wherein the viscosity and adhesive strength are adjusted in the above range. By this blend ratio are greatly improved the adhesive strength of the obtained base, sticking properties to the skin, a uniform dispersion of a drug in the base, a pain at the time of release, a damage toward the horny layer, a rash on the skin, a thermal stability and the like.

The base of the patch of the invention may contain a filler, an antioxidant, an ultraviolet absorbent, a resolvent and the like. As fillers, zinc oxide, aluminumoxide, titaniumdioxide, calcium carbonate, synthetic aluminum silicate, silica, magnesium oxide, metal salts of stearic acid and the like are used. As antioxidants, for example, ascorbic acid, tocopherol acetate, natural vitamin E, dibutyl hydroxytoluene, propyl gallate and the like are used. As ultraviolet absorbents, for example, 2-hydroxy-4-methoxybenzophenone, glycol salicylate, 2-(2-hydroxy-5-methylphenyl)benzotriazole and the like are used, though it is preferable not to be contained considering the safety for the skin. As resolvents, for example, oleic acid, benzyl alcohol, isopropylmyristate, crotamiton, oleyl alcohol, eucalyptus oil, limonene, isopulegol or other oils are used. In addition, a surfactant, a fat, a higher fatty acid, a flavoring agent and the like may be contained if necessary. Further, a skin irritant (counterirritant) such as L-menthol, camphor, mentha oil, red pepper extract, capsaicin, benzyl nicotinate, methyl salicylate or glycol salycilate can appropriately be blended if necessary.

In the following, a preparation method of the patch of the invention is explained. As one example, first, a tackifier and a plasticizer are added to styrene-isoprene-styrene block copolymer and polyisobutylene to adjust the viscosity and adhesive strength, followed optionally by addition of a filler, an antioxidant and the like at a designated ratio to give a mixture. The mixture is heated under stirring in an atmosphere of nitrogen to give a melt. The temperature at the time of stirring is 110-200°C, and the stirring period is 30-120 min. Then, an effective ingredient is added to the above melt under stirring at 110-200°C and mixed for 1-30 min to give a homogeneous melt. Next, the melt is directly spreaded on a backing which is specially treated with an ultraviolet absorbent and/or an ultraviolet-shielding agent in a usual manner, then covered with a releasable coat, or otherwise, after once spreading on the releasable coat, a pressure transfer may be made by covering with the backing. The releasable coat is appropriately selected from a release paper which is carried out with a release treatment, a cellophane, or a film such as polyethylene, polypropylene or polyester.

### [Example]

In the following, the invention is explained in more detail by the examples. The invention, however, is not limited to these examples, and various changes may be made without departing from the spirit of the invention. Further, in the examples, all % mean % by mass.

### Tape Preparation 1 (Example 1)

Styrene-isoprene-styrene block copolymer (KRATON D-1107CU: manufactured by Shell Chemical) of 22 parts by mass, polyisobutylene (Oppanol B80: manufactured by BASF) of 22 parts by mass, hydrogenated rosin ester (Staybelite Ester: manufactured by Rika Hercules) of 12 parts by mass, liquid paraffin (Crystol J-325; manufactured by Esso Petroleum) of 40 parts by mass and dibutyl hydroxytoluene of 1 part of mass were heated at 110-200°C under a nitrogen atmosphere, added with ketoprofen of 3 parts by mass under stirring, and further mixed for 5-30 min to obtain a homogeneous melt which was made a tape base. Then, the base was spreaded on a polyester film treated with silicone to become 1 g per 70cm².

In the meantime, 2-(3-t-butyl-5-methyl-2-hydroxyphenyl)-5-chlorobenzotriazole (TINUVIN326: manufactured by Nagase Kasei) of 1 part by mass was adsorbed to a polyester woven fabric of 99 parts by mass, in which the weight is about 110g/cm², to obtain a backing with an ultraviolet-shielding treatment. The spreaded base on the above film was covered by this backing and allowed to a pressure transfer to give the tape preparation by cut in a desirable size.

### Tape Preparation 2 (Example 2)

The ketoprofen tape preparation was obtained in the same way as that of the example 1 except adsorbing 2-(3-t-butyl-5-methyl-2-hydroxyphenyl)-5-chlorobenzotriazol e (TINUVIN326: manufactured by Nagase Kasei) of 2 parts by mass to the polyester woven fabric of 98 parts by mass and obtaining a backing with an ultraviolet-shielding treatment.

### Tape Preparation 3 (Example 3)

The ketoprofen tape preparation was obtained in the same way as that of the example 1 except adsorbing 2-(3-t-butyl-5-methyl-2-hydroxyphenyl)-5-chlorobenzotriazol e (TINUVIN326: manufactured by Nagase Kasei) of 3 parts by mass to the polyester woven fabric of 97 parts by mass and obtaining a backing with an ultraviolet-shielding treatment.

### Tape Preparation 4 (Example 4)

The ketoprofen tape preparation was obtained in the same way as that of the example 1 except adsorbing 2-(3-t-butyl-5-methyl-2-hydroxyphenyl)-5-chlorobenzotriazol e (TINUVIN326: manufactured by Nagase Kasei) of 1 part by mass to a woven fabric in which a polyester resin of 97.5 parts by mass was incorporated with titanium oxide of 1.5 parts by mass, and obtaining a backing with an ultraviolet-shielding treatment.

### Tape Preparation 5 (Example 5)

The ketoprofen tape preparation was obtained in the same way as that of the example 1 except adsorbing 2-(3-t-butyl-5-methyl-2-hydroxyphenyl)-5-chlorobenzotriazol e (TINUVIN326: manufactured by Nagase Kasei) of 2 parts by mass to a woven fabric in which a polyester resin of 96.5 parts by mass was incorporated with titanium oxide of 1.5 parts by mass, and obtaining a backing with an ultraviolet-shielding treatment.

### Tape Preparation 6 (Example 6)

The ketoprofen tape preparation was obtained in the same way as that of the example 1 except adsorbing 2-(3-t-butyl-5-methyl-2-hydroxyphenyl)-5-chlorobenzotriazol e (TINUVIN326: manufactured by Nagase Kasei) of 2 parts by mass to a woven fabric in which a polyester resin of 97.9 parts by mass was incorporated with titanium oxide of 0.1 parts by mass, and obtaining a backing with an ultraviolet-shielding treatment.

### Tape Preparation 7 (Example 7)

The ketoprofen tape preparation was obtained in the same way as that of the example 1 except adsorbing 2-(3-t-butyl-5-methyl-2-hydroxyphenyl)-5-chlorobenzotriazol e (TINUVIN326: manufactured by Nagase Kasei) of 2 parts by mass to a woven fabric in which a polyester resin of 97.8 parts by mass was incorporated with titanium oxide of 0.2 parts by mass, and obtaining a backing with an ultraviolet-shieldingtreatment.

### Tape Preparation 8 (Comparative examples 1, 3)

The ketoprofen tape preparation was obtained in the same way as that of the example 1 except no ultraviolet-shielding treatment to the polyester woven fabric (no treatment).

### Tape Preparation 9 (Comparative example 2)

The ketoprofen tape preparation was obtained in the same way as that of the example 2 except changing the ultraviolet absorbent used in the ultraviolet-shielding treatment for the polyester woven fabric into

### 2-(2'-hydroxy-5'-methylphenyl)benzotriazole.

### Photo-transmission test

As for each backing used in the tape preparations 1-9 (Examples 1-7 and Comparative examples 1-3), the photo-transmission tests were carried out. First, an ultraviolet intensity was measured under a direct sunlight by an ultraviolet intensity meter (Topcon Co., Ltd., UVR-2). At this time, UD-36 was used for a receiving part, and the measuring wave length was 310-400nm. Then, an ultraviolet dose transmitting through the backing was measured under a circumstance in which a direct sunlight irradiated enough to each backing, and when the ultraviolet intesity (Examples 1-5, Comparative examples 1 and 2, about 3.0 mW/cm²; Examples 6-7, Comparative example 3, about 1.6 mW/cm²) without the above backing was made 100, the ultraviolet transmittances were calculated for the photo-transmission tests.

### Photo-stability test 1

As for each of the tape preparations 1-9 (Examples 1-7 and Comparative examples 1-3), the photo-stability test of the drug was carried out. Namely, the backing surface of each tape preparation was turned upward and let stand at a place where a direct sunlight irradiated enough, whereby a drug remaining ratio in the base was measured by liquid chromatography. Further, a daytime (fine weather) ultraviolet dose in Tosu-shi, Saga prefecture on August 22, 2002 when the tests of Examples 1-5 and Comparative examples 1 and 2 were carried out was about 10000mJ/cm² per hour, the irradiation time being 8 hours (accumulatedultraviolet dose, about 80000mJ/cm²), and a daytime ultraviolet dose in Tosu-shi, Saga prefecture on November 5-6, 2002 when the tests of Examples 6 and 7 and Comparative example 3 were carried out was about 6000mJ/cm² per hour, the irradiation time being 15 hours (accumulated ultraviolet dose, about 85000mJ/cm²) .

### Photo-stability test 2

The backing surface of each tape preparation was turned upward and let stand at a place where a direct sunlight irradiated enough, and as to the degree of coloring of the base after the tests (Examples 1-5, Comparative examples 1 and 2: ultraviolet dose, about 10000mJ/cm² per hour; irradiation time, 8 hours; accumulated ultraviolet dose, about 80000mJ/cm², Examples 6 and 7, Comparative example 3: ultraviolet dose, about 6000mJ/cm² per hour; irradiation time, 15 hours; accumulated ultraviolet dose, about 85000mJ/cm²), the external appearance was observed.

### Photo-stability test 3

The backing surface of each tape preparation was turned upward and let stand at a place where a direct sunlight irradiated enough, and as to the agglutinative strength (stickiness) of the base after the tests (Examples 1-5, Comparative examples 1 and 2 : ultraviolet dose, about 10000mJ/cm² per hour; irradiation time, 8 hours; accumulated ultraviolet dose, about 80000mJ/cm², Examples 6 and 7, Comparative example 3: ultraviolet dose, about 6000mJ/cm² per hour; irradiation time, 15 hours; accumulated ultraviolet dose, about 85000mJ/cm²), the external appearance was observed.

The results are summarized in Table 1.

**[Table 1]**

| | Tape preparations | Drug content after test (%) | Ultraviolet transmittance (%) | Appearance change | Degradation of adhesive mass (stickiness) |
|---|---|---|---|---|---|
| Example 1 | Formula 1 | 90.8 | 1.92 | no | no |
| Example 2 | Formula 2 | 98.7 | 1.38 | no | no |
| Example 3 | Formula 3 | 99.9 | 0.67 | no | no |
| Example 4 | Formula 4 | 98.8 | 0.84 | no | no |
| Example 5 | Formula 5 | 100.0 | 0.44 | no | no |
| Example 6 | Formula 6 | 99.3 | 0.84 | no | no |
| Example 7 | Formula 7 | 99.5 | 0.67 | no | no |
| Comparative Example 1 | Formula 8 | 53.0 | 24.8 | yellow | yes |
| Comparative Example 2 | Formula 9 | 77.5 | 2.83 | faint yellow | partly yes |
| Comparative Example 3 | Formula 8 | 49.8 | 24.8 | yellow | yes |

### Sensory test

The tape preparation 5 (70cm²) was adhered to knees of five volunteers for 8 hours, and the sticking properties and the uncomfortable feeling when sticking were evaluated to carry out the total judgment.

### Evaluation standard for useability

### Sticking properties

No peeling off: ⊚ End part was peeled off: O Not less than 1/4 were peeled off: Δ Not less than 1/2 were peeled off: ×
Uncomfortable feeling when sticking
No tightening feeling: O Slight tightening feeling: Δ
Tightening feeling: ×
Total judgment
Satisfied with useablity: O Partly dissatisfied with useablity: Dissatisfied with useablity: ×

The results were summarized in Table 2.

**[Table 2]**

| | Weight (g/m²) | Ultraviolet transmittance (%) | Useability | | |
|---|---|---|---|---|---|
| | | | Sticking properties | Uncomfortable felling when sticking | Total judgment |
| Tape preparation | 112.5 | 0.44 | ⊚ | ○ | ○ |

### [Industrial Applicability]

The patch of the invention is excellent in an ultraviolet-shielding effect, can lower an ultraviolet transmittance of a backing even when exposed to the direct sunlight with a high ultraviolet dose, does not produce degradation such as oozing of a pressure-sensitive adhesive layer, etc., and can exhibit satisfactory drug effects. In addition, since an ultraviolet absorbent itself does not contact to the skin, irritation to the skin can be lowered, and therefore, the safety and also the use feeling are excellent.

## Claims

1. A patch comprising a polyester backing and a pressure-sensitive adhesive layer formed on one surface of the backing and containing a nonsteroidal anti-inflammatory drug (NSAID) wherein the backing contains a hydroxyphenylbenzotriazole derivative represented by the general formula (1): 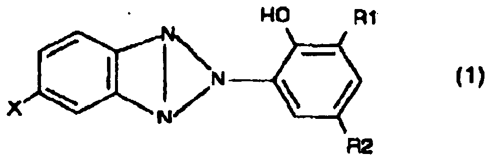 wherein R₁ and R₂ are each independently hydrogen or C₁₋₈ alkyl; and X is halogen atom, and wherein the nonsteroidal anti-inflammatory drug (NSAID) is ketoprofen.

2. The patch according to claim 1, wherein the backing contains further titanium oxide.

3. The patch according to claim 1 or 2, wherein the ultraviolet transmittance of the backing is not more than 2%.

4. The patch according to claim 3, wherein the ultraviolet transmittance of the backing is not more than 1.5%.

5. The patch according to any one of claims 1-4, wherein the weight of the backing is 100g/m² - 130g/m².

6. The patch according to any one of claims 1-5, wherein the pressure-sensitive adhesive layer consists of a styrene-isoprene-styrene block copolymer and/or polyisobutylene.

7. The patch according to any one of claims 1-6, wherein the pressure-sensitive adhesive layer contains no ultraviolet absorbent.

## Patentansprüche

1. Pflaster umfassend einen Polyester-Träger und eine Haftkleberschicht, welche auf einer Oberfläche des Trägers gebildet ist und ein nichtsteroidales entzündungshemmendes Medikament (NSAID) enthält, wobei der Träger ein durch die allgemeine Formel (1) dargestelltes Hydroxyphenylbenzotriazol-Derivat enthält: 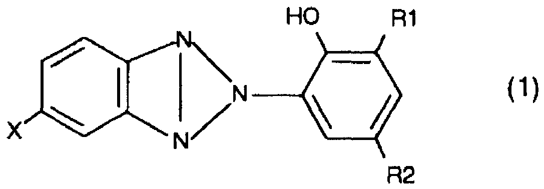 wobei R₁ und R₂ jeweils unabhängig voneinander Wasserstoff oder C₁₋₈-Alkyl sind; und X ein Halogenatom ist, und wobei das nichtsteroidale entzündungshemmende Medikament (NSAID) Ketoprofen ist.

2. Pflaster nach Anspruch 1, wobei der Träger weiterhin Titanoxid umfasst.

3. Pflaster nach Anspruch 1 oder 2, wobei die Ultraviolett-Transmission des Trägers nicht mehr als 2% beträgt.

4. Pflaster nach Anspruch 3, wobei die Ultraviolett-Transmission des Trägers nicht mehr als 1.5% beträgt.

5. Pflaster nach einem der Ansprüche 1-4, wobei das Gewicht des Trägers 100g/m² - 130g/m² beträgt.

6. Pflaster nach einem der Ansprüche 1-5, wobei die Haftkleberschicht aus einem Styrol-Isopren-Styrol-Blockcopolymer und/oder Polyisobutylen besteht.

7. Pflaster nach einem der Ansprüche 1-6, wobei die Haftkleberschicht kein Ultraviolett-Absorbens enthält.

## Revendications

1. Patch comprenant un support en polyester et une couche adhésive sensible à la pression réalisée sur une surface du support et contenant un anti-inflammatoire non stéroïdien (AINS) où le support contient un dérivé d'hydroxyphényl benzotriazole représenté par la formule générale (1): 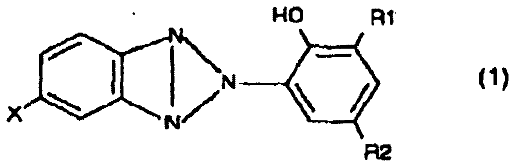 où R₁ et R₂ représentent chacun indépendamment de l'hydrogène ou alkyle C₁₋₈ ; et X est un atome halogène, et où l'anti-inflammatoire non stéroïdien (AINS) est kétoprofène.

2. Patch selon la revendication 1, où le support contient en outre de l'oxyde de titane.

3. Patch selon la revendication 1 ou 2, où le facteur de transmission d'ultraviolets du support n'est pas supérieur à 2%.

4. Patch selon la revendication 3, où le facteur de transmission d'ultraviolets du support n'est pas supérieur à 1,5%.

5. Patch selon l'une des revendications 1 à 4, où le poids du support est entre 100 g/m² et 130 g/m².

6. Patch selon l'une des revendications 1 à 5, où la couche adhésive sensible à la pression consiste en un copolymère séquencé styrène/isoprène et/ou polyisobutylène.

7. Patch selon l'une des revendications 1 à 6, où la couche adhésive sensible à la pression ne contient pas d'absorbant d'ultraviolets.
